# FASCICULE DE BREVET EUROPEEN

(11) **EP 2 189 180 B1**
(45) Date de publication et mention de la délivrance du brevet: **08.06.2011**
(21) Numéro de dépôt: 09173833.6
(22) Date de dépôt: 22.10.2009
(51) Int. Cl.: A61N 1/368, A61N 1/39

(54) **Dispositif médical actif implantable comprenant des moyens de calcul du délai atrioventriculaire**
Aktives medizinisches Implantat, das mit Berechnungsmitteln der atrioventrikulären Verzögerung ausgestattet ist
Implantable active medical device comprising means for calculating atrioventricular delay

(30) Priorité: 19.11.2008 FR 0806462
(43) Date de publication de la demande: 26.05.2010
(73) Titulaire: ELA MEDICAL, 92541 Montrouge (FR)
(72) Inventeur: Casset, Cyrille, 33650, SAINT SELVE (FR)
(74) Mandataire: Dupuis-Latour, Dominique

(56) Documents cités:
- WO-A-2005/089866
- US-A1- 2006 293 715
- US-A1- 2007 179 541

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle s'applique plus particulièrement à ceux de ces dispositifs qui possèdent au moins une sonde de détection/stimulation de l'oreillette ou des deux oreillettes. Il s'agit principalement des implants de type stimulateur ou défibrillateur double chambre atrio-ventriculaire ou bien de type "multi-site" triple ou quadruple chambre.

En fonctionnement "double chambre", à la suite d'un événement auriculaire, qu'il s'agisse d'une dépolarisation spontanée (onde P détectée) ou bien stimulée (application d'une impulsion A), le dispositif surveille l'activité ventriculaire et, en même temps, commence à compter un délai dit "délai atrio-ventriculaire" ou "délai auriculo-ventriculaire", généralement désigné DAV ou AVD. Si, à l'issue de ce délai aucune activité spontanée ventriculaire (onde R) n'a été détectée, alors le dispositif déclenche une stimulation de ce ventricule (application d'une impulsion V).

L'invention peut s'appliquer notamment, bien que de façon non limitative, aux dispositifs comprenant des circuits de stimulation et de détection à la fois sur l'oreillette et sur le ventricule et qui peuvent opérer selon deux modes de fonctionnement, DDD ou AAI : au départ, le mode de fonctionnement du stimulateur est le mode AAI avec surveillance de l'activité ventriculaire ; l'algorithme recherche alors la présence ou l'absence d'une activité ventriculaire, qui dans ce cas pourrait laisser suspecter un bloc auriculo-ventriculaire (BAV), de manière à éventuellement basculer en mode DDD de stimulation double chambre avec association atrio-ventriculaire, c'est-à-dire avec calcul et application d'un DAV pour la stimulation contrôlée du ventricule (mode dénommé AAlSafeR").

Les EP 0 488 904 A1 et EP 1 346 750 A1 (tous deux au nom de ELA Medical) décrivent de tels dispositifs à commutation automatique du mode de fonctionnement AAI/DDD.

Dans tous les cas, il est important de définir de façon précise la durée du DAV.

En effet, du point de vue de la mécanique cardiaque, le DAV doit être suffisamment long pour permettre à l'oreillette de se contracter complètement et par là même de vider dans le ventricule le sang qu'elle contient, et la contraction ventriculaire doit en conséquence se produire après que la contraction atriale ait entièrement eu lieu. Mais elle ne doit pas survenir trop longtemps après, car un DAV trop long risquerait de dissocier le système oreillette/ventricule, avec risque de déclencher des arythmies par conduction rétrograde, ou de réduire l'efficacité hémodynamique du cycle cardiaque : comme la contraction auriculaire termine le remplissage ventriculaire, le délai entre la fin de ce remplissage et le début de la vidange du ventricule constitue un temps mort, "perdu" sur le plan hémodynamique. De plus, tout délai prolongé de DAV impacte sur la diastole ventriculaire (remplissage du ventricule post-vidange) et donc "recule" d'autant la fin de ce remplissage ventriculaire, qui se superpose alors à la contraction atriale suivante.

Il est donc important de pouvoir adapter au mieux le DAV pour chaque patient, de manière que le début de la vidange ventriculaire (provoquée par la stimulation du ventricule) intervienne tout de suite après la fin du remplissage du ventricule par l'oreillette.

Dans la plupart des dispositifs, le DAV est ajusté automatiquement en fonction de la fréquence sinusale détectée, le DAV pouvant prendre diverses valeurs entre une valeur maximale (DAV de base) et une valeur minimale. La valeur du DAV calculée à partir de la fréquence est en outre augmentée d'une période additionnelle si l'événement auriculaire est un événement stimulé, de manière à compenser le délai électro-mécanique induit par une stimulation atriale non native du noeud sinusal.

Les paramètres de base du calcul automatique du DAV sont programmés par le praticien au moment de l'implantation, ou à l'occasion de visites de suivi du patient. Ils peuvent être éventuellement réajustés automatiquement par le dispositif *a posteriori*, après analyse du rythme cardiaque du patient sur une période longue.

La programmation de ces paramètres ne prend toutefois pas en compte la réalité hémodynamique propre à chaque patient, et de plus ne permet pas un ajustement fin du DAV, car le calcul de ce DAV ne prend pas en compte le point de savoir si la contraction atriale s'est ou non déroulée entièrement avant que la stimulation ventriculaire ne soit déclenchée.

Pour remédier à cette difficulté, le WO 2005/089866 A1 propose de détecter la contraction de l'oreillette par un signal d'accélération endocardiaque délivré par un capteur accélérométrique approprié. Un tel capteur peut être présent sur la sonde auriculaire, ou sur une autre sonde possédant un tel capteur, placée soit directement dans l'oreillette, soit dans une autre position permettant de recueillir le signal d'accélération endocardiaque (signal EA) représentatif des contractions de l'oreillette.

Dans cette autre approche le dispositif, après avoir stimulé l'oreillette, utilise un signal fonctionnel - le signal EA - représentatif de la mécanique cardiaque, à la place d'un signal issu de la propagation électrique de l'onde de dépolarisation. Ce signal mécanique peut également être exploité en complément du signal électrique, comme le US2007/0179541 A1 qui propose de mesurer et d'analyser le retard entre la détection électrique et la détection mécanique de la contraction atriale.

Le WO 2005/089866 A1 suggère d'utiliser le signal EA à diverses fins telles qu'optimisation du délai atrioventriculaire DAV dans le cas d'une stimulation double chambre, optimisation du délai interventriculaire DVV dans le cas d'une stimulation biventriculaire pour une thérapie de resynchronisation CRT, détection de capture dans une cavité cardiaque, etc.

Toutefois, comme pour le signal électrique, la composante du signal EA correspondant à l'activité de l'oreillette présente non seulement une amplitude beaucoup plus faible que dans le cas du ventricule, mais elle est en outre beaucoup plus précoce, ce qui rend sa détection et son analyse beaucoup plus difficile. C'est pour cette raison qu'il n'a jusqu'à présent pas été proposé de technique basée sur l'analyse de la composante atriale du signal EA qui procure des résultats réellement exploitables, malgré l'intérêt de disposer d'un signal reflétant directement l'activité mécanique du coeur.

L'un des buts de l'invention est de remédier à cette difficulté, en proposant un dispositif comprenant des moyens de calcul du DAV permettant de suivre directement le déroulement du cycle cardiaque du point de vue mécanique, afin d'être certain que la contraction de l'oreillette soit effectivement achevée lorsque l'impulsion de stimulation du ventricule sera délivrée au coeur.

La présente invention se base sur l'approche consistant à détecter mécaniquement la contraction auriculaire, notamment le moment auquel cette contraction prend fin, par le biais d'un signal d'accélération endocardiaque (signal EA) délivré par un capteur accélérométrique approprié.

En effet, diverses études cliniques ont montré que l'accélération endocardiaque est un paramètre permettant de fournir des informations très complètes sur la mécanique cardiaque, aussi bien dans le cas d'un fonctionnement normal que d'un fonctionnement déficient. L'accélération endocardiaque, qui est mesurée par un accéléromètre couplé au muscle cardiaque, reflète très précisément et en temps réel les phénomènes liés aux mouvements de la cavité cardiaque (la cavité auriculaire dans le cas de la présente invention).

Ainsi, le EP 0 515 319 A1 (Sorin Biomedica Cardio SpA) enseigne la manière de recueillir un signal EA au moyen d'une sonde endocavitaire intégrant un micro-accéléromètre permettant de mesure l'accélération endocardiaque.

On notera toutefois que, bien que dans la présente invention on fasse principalement référence à l'analyse d'un signal EA délivré par un capteur implanté (typiquement, un capteur placé sur une sonde endocavitaire) l'invention est également applicable à une analyse opérée à partir d'un signal EA externe obtenu de manière non invasive. Un tel signal EA peut être par exemple un signal issu d'un capteur fixé sur la poitrine du patient au niveau du sternum. Ici et dans la suite, lorsque l'on parlera de "signal EA", ce terme sera entendu comme couvrant aussi bien un signal EA externe, obtenu de manière non invasive, qu'un signal EA endocavitaire, obtenu par un capteur d'accélération monté sur une sonde introduite dans une cavité du coeur du patient, ou encore un signal EA délivré par une sonde implantée épicardique en contact direct avec le myocarde.

Essentiellement, l'invention propose donc, après avoir détecté par un signal électrique la dépolarisation correspondant à un événement auriculaire (spontané ou stimulé), d'utiliser un signal fonctionnel représentatif de la mécanique cardiaque (le signal EA), pour calculer un DAV tel que la contraction de l'oreillette soit effectivement achevée, et juste achevée, à l'issue de ce DAV. Ceci au lieu de calculer le DAV sur la base de paramètres préprogrammés de façon plus ou moins empirique, ou recalculés directement à partir de l'analyse du rythme et du séquencement atrial et ventriculaire.

Plus précisément, l'invention propose un dispositif du type générique connu, par exemple d'après le WO 2005/089866 A1 précité, comportant : des moyens de détection d'événements auriculaires ; des moyens de détection d'événements ventriculaires ; des moyens pour calculer un délai atrio-ventriculaire DAV et pour débuter ce DAV sur détection d'un événement auriculaire spontané ou stimulé ; et des moyens de stimulation ventriculaire, aptes à délivrer à l'expiration du DAV une impulsion de faible énergie en l'absence d'événement ventriculaire spontané détecté. Les moyens de calcul de DAV comprennent : un capteur d'accélération, apte à délivrer un signal EA représentatif des mouvements produits par les contractions cycliques de la cavité auriculaire ; et des moyens d'analyse de signal EA, aptes à reconnaître et isoler dans le signal EA délivré par le capteur une composante EA4 correspondant au quatrième pic d'accélération endocardiaque associé à l'activité auriculaire, et à calculer la valeur du DAV à partir de la composante EA4.

De façon caractéristique de l'invention, les moyens d'analyse de signal EA sont des moyens aptes à déterminer un paramètre de la composante EA4 fonction de l'instant de fin de la composante EA4, et à calculer la valeur du DAV en fonction dudit paramètre.

Le DAV peut être calculé de manière que la fin du DAV soit concomitante ou postérieure à l'instant de fin de la composante EA4, la fin du DAV étant en particulier définie par l'instant de fin de la composante EA4.

Dans un perfectionnement avantageux, les moyens d'analyse de signal EA sont également aptes à reconnaître et isoler dans le signal EA délivré par le capteur une composante EA1 correspondant au premier pic d'accélération endocardiaque associé à l'activité auriculaire, et à déterminer un instant de début de cette composante EA1. La valeur du DAV est alors calculée en fonction également de l'instant de début de la composante EA1.

Le DAV peut notamment être calculé en fonction de l'intervalle de temps séparant l'instant de fin de la composante EA4 de l'instant de début de la composante EA1 immédiatement consécutive, ou bien de manière à faire concorder l'instant de fin de la composante EA4 avec l'instant de début de la composante EA1 immédiatement consécutive, ou encore de manière que l'instant de fin de la composante EA4 précède d'une durée prédéterminée l'instant de début de la composante EA1 immédiatement consécutive.

Dans une forme de mise en oeuvre préférentielle, les moyens d'analyse de signal EA comprennent des moyens pour quantifier un paramètre du signal EA à l'intérieur d'au moins une fenêtre temporelle d'analyse de durée prédéterminée, cette fenêtre étant déclenchée après l'instant d'application de l'impulsion de stimulation auriculaire, et se terminant sur autre événement spécifique, tel que : détection ventriculaire, stimulation ventriculaire, apparition d'un signal EA1, ou délai fixe.

Le paramètre quantifié peut être en particulier l'énergie du signal EA, intégrée sur la durée de la fenêtre d'analyse, et ce paramètre est quantifié à l'intérieur d'une pluralité de fenêtres temporelles d'analyse successives, notamment des fenêtres chevauchantes.

Il peut être notamment prévu des moyens comparateurs, pour comparer à un seuil donné le paramètre du signal EA quantifié à l'intérieur de la fenêtre temporelle, l'instant de fin de la composante EA4 étant alors défini par le franchissement à la baisse de ce seuil. Ce seuil peut être un seuil fixe, un seuil paramétrable, un seuil adaptatif recalculé à intervalles réguliers, ou encore un seuil adaptatif recalculé sur chaque cycle comprenant un événement auriculaire valide.

Le capteur d'accélération peut être un capteur endocavitaire, un capteur épicardique ou encore un capteur externe.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre un exemple de signal d'accélération endocardiaque EA recueilli au cours de trois cycles cardiaques successifs.
La Figure 2 est une série de trois chronogrammes illustrant différents signaux caractérisant l'activité cardiaque au cours d'un cycle donné.
La Figure 3 est une série de quatre chronogrammes montrant la manière dont est opérée la détection de la composante représentative PEA4 selon un mode de réalisation préférentiel de l'invention.
La Figure 4 est une série de trois chronogrammes montrant un exemple de signal d'accélération endocardiaque EA obtenu, respectivement, dans le cas d'un DAV trop long, d'un DAV correctement ajusté et d'un DAV trop court.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par ELA Médical, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

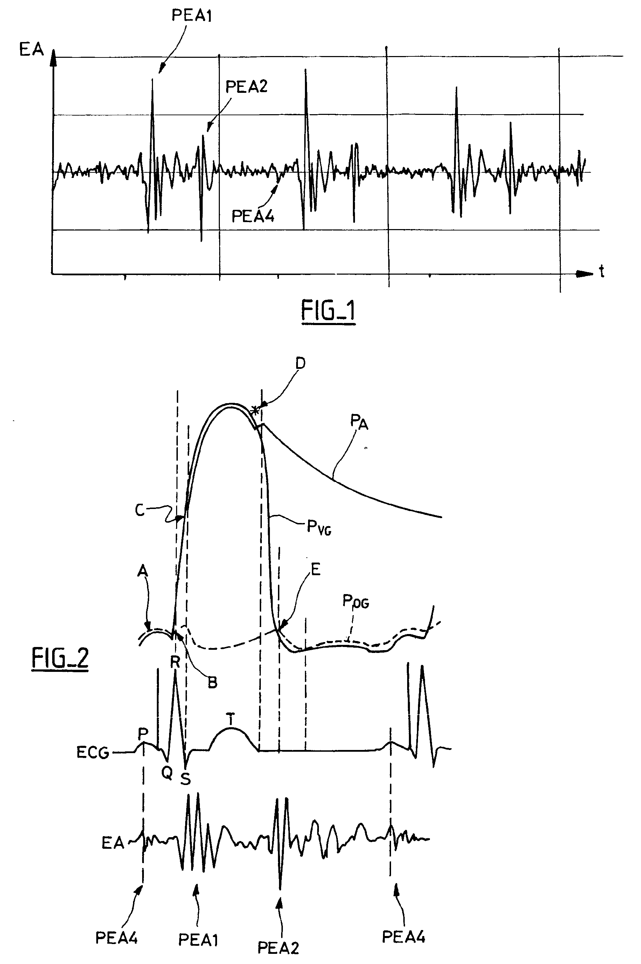

Comme illustré sur la Figure 1, qui est un exemple de signal d'accélération endocardiaque (EA) recueilli au cours de trois cycles cardiaques successifs, le signal EA forme au cours d'un cycle cardiaque deux pics principaux, correspondant aux deux bruits majeurs (sons S1 et S2 du phonocardiogramme) qu'il est possible de reconnaître dans chaque cycle d'un coeur sain :
- le premier pic d'accélération endocardiaque ("PEA1 "), dont les variations sont étroitement liées aux variations de la pression dans le ventricule (l'amplitude du pic PEA1 étant, plus précisément, corrélée au maximum positif de la variation de pression dP/dt dans le ventricule gauche) ;
- le second pic d'accélération endocardiaque ("PEA2"), quant à lui, correspond à la phase de relaxation ventriculaire isovolumique et il est produit par la décélération brusque de la masse sanguine en mouvement dans l'aorte.

Les composantes EA1 et EA2 du signal EA sont celles qui correspondent aux deux pics d'accélération endocardiaque respectifs PEA1 et PEA2.

Le signal EA contient également deux autres composantes, d'amplitude très inférieure, appelées EA3 et EA4, correspondant aux sons S3 et S4 du phonocardiogramme.

L'invention s'intéresse plus particulièrement à la composante EA4, qui est directement liée à la présence d'une contraction auriculaire.

Essentiellement, l'invention se propose d'exploiter cette composante auriculaire afin de gérer les paramètres d'un stimulateur lié à l'activité auriculaire.

Cette composante présente en particulier un pic (ci-après "PEA4") qui, comme on peut le voir sur la Figure 1, se situe immédiatement avant le pic PEA1 - et pour cette raison ce pic est quelquefois désigné "PEA0" par les rythmologues, dans la mesure où, du point de vue électrique, la contraction de l'oreillette précède la contraction du ventricule. En revanche, si l'on se place du point de vue du flux sanguin pompé par le myocarde, la contraction de l'oreillette (correspondant à la composante EA4) achève le remplissage du ventricule en fin de diastole (composante EA2) et se situe donc, du point de vue de l'hémodynamique cardiaque, après cette dernière - d'où la désignation "PEA4".

La Figure 2 illustre les différents signaux caractérisant l'activité du coeur au cours d'un cycle cardiaque, avec : le profil des pressions intracardiaques, un relevé d'électrocardiogramme de surface (ECG), et les variations du signal d'accélération endocardiaque (EA).

Sur le profil des pressions intracardiaques, la caractéristique P_{A} illustre les variations de la pression aortique, P_{VG} celles du ventricule gauche et P_{OG} celles dans l'oreillette gauche. Les points A à E correspondent aux différentes phases suivantes : A, contraction de l'oreillette gauche ; B, fermeture de la valvule mitrale ; C, ouverture de la valvule aortique ; D, fermeture de la valvule aortique ; et E, ouverture de la valvule mitrale.

Le signal ECG comprend successivement : l'onde P correspondant à la dépolarisation de l'oreillette, le complexe QRS correspondant à la dépolarisation du ventricule et l'onde T de repolarisation ventriculaire.

Le signal d'accélération endocardiaque EA, quant à lui, peut se décomposer de la manière suivante : la composante EA4 correspond à la contraction de l'oreillette (onde P), et elle est suivie par la composante EA1, qui débute à la suite du complexe QRS et est engendrée par une combinaison de la fermeture des valves auriculo-ventriculaires, de l'ouverture des valves semi-lunaires et de la contraction du ventricule gauche. La composante EA2 qui lui fait suite, quant à elle, accompagne la fin de la systole ventriculaire et elle est générée par la fermeture des valves semi-lunaires.

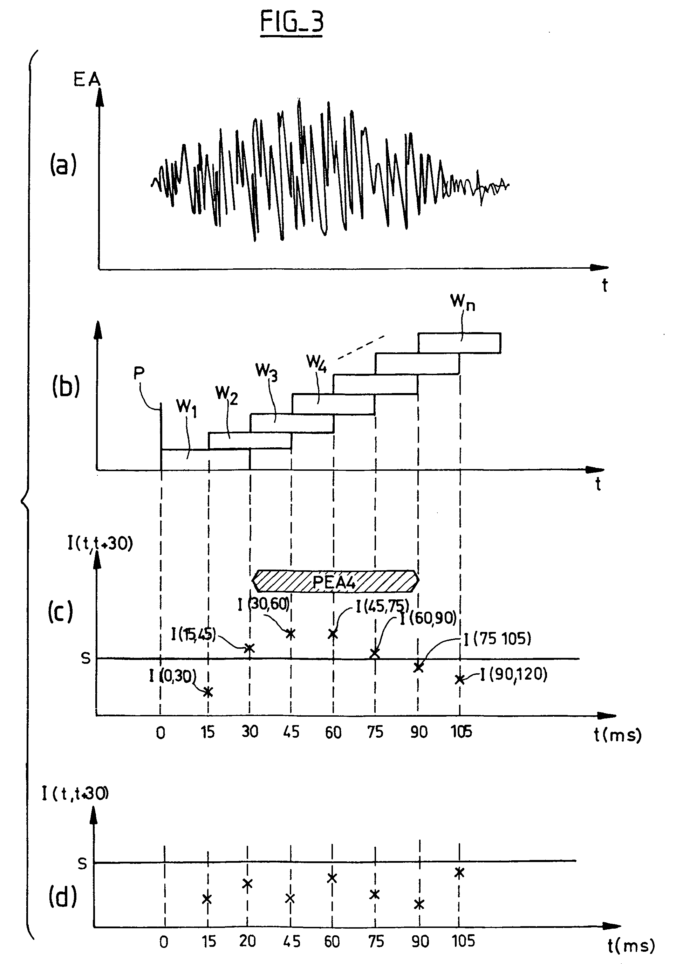

La Figure 3 illustre, sous forme d'une série de chronogrammes, un exemple avantageux de mise en oeuvre de la présente invention pour la détection de la composante EA4, permettant notamment (i) de détecter la présence ou non d'un pic PEA4 et (ii) dans l'affirmative, de déterminer les instants de début et de fin de ce pic.

Le chronogramme de la Figure 3a illustre le signal d'accélération endocardiaque EA dans la période qui suit immédiatement la stimulation auriculaire, événement indiqué par le marqueur P sur le chronogramme de la Figure 3b.

Cet événement P déclenche une première fenêtre temporelle W1 (Figure 3b), par exemple d'une durée de 30 ms. Sur la durée de cette fenêtre est calculé un indice I(0,30) représentatif de la puissance du signal EA sur l'intervalle 0-30 ms. Cet indice est par exemple déterminé à partir des valeurs numérisées du signal échantillonné, en calculant l'intégrale de la valeur absolue du ce signal EA sur cet intervalle.

La valeur de l'indice I(0,30) qui représente donc la puissance moyenne du signal EA sur la durée de la fenêtre W1 centrée sur l'instant t = 15 ms, est reportée par un point représenté sur le chronogramme de la Figure 3c à l'abscisse t = 15 ms.

Cette détermination est réitérée pour une nouvelle fenêtre W2, décalée par rapport à la fenêtre W1 par exemple de 15 ms (le décalage t = 15 ms correspondant à la durée d'une fenêtre, divisée par deux). Cette seconde détermination, effectuée donc sur l'intervalle t = 15-45 ms, donne un nouvel indice I(15,45).

La détermination est à nouveau réitérée, sur une succession de fenêtres glissantes W3, W4, ... Wn, chacune décalée de 15 ms par rapport à la fenêtre précédente.

La réitération est poursuivie par exemple jusqu'à détection d'un événement ventriculaire, ou jusqu'à ce que l'indice I(t,t+30) passe en dessous d'un seuil limite, ou encore après écoulement d'une période fixe limite, typiquement d'une centaine de millisecondes.

Sur la base de l'évaluation que l'on vient de décrire, on considère qu'il y a contraction auriculaire sur le cycle cardiaque en question si l'indice I(t,t+30) est, pour au moins l'une des fenêtres, supérieur à un seuil prédéterminé S (Figure 3c).

La définition de ce seuil S peut être arbitraire, ou adaptée au patient (paramétrable par le praticien) ou bien encore résulter d'un calcul adaptatif remis à jour régulièrement. Comme exemple particulier de seuil adaptatif, on peut considérer un événement auriculaire spontané (dépolarisation non stimulée de l'oreillette) et calculer les indices I(0,30), I(15,45), I(30,60), ... sur une période donnée. Le seuil est ensuite défini comme valant 50 % de la valeur maximale de toutes les valeurs d'indice ainsi calculées. Le seuil peut être recalculé à intervalles réguliers, typiquement une fois par jour, ou bien sur chaque événement auriculaire validé par le dispositif.

Si le test ainsi effectué permet d'avérer la présence d'une contraction auriculaire, le dispositif détermine les instants de début et de fin du pic d'accélération endocardiaque de la composante EA4 (PEA4), par exemple en considérant que le pic s'étend de la première valeur d'indice I(t,t+30) dépassant le seuil jusqu'à la première valeur d'indice I(t,t+30) située au-dessous de ce même seuil, comme illustré sur la Figure 3c.

L'instant de fin du pic d'accélération endocardiaque de la composante EA4 peut être recherché de façon plus fine avec une succession de fenêtres glissantes décalées chacune de 5 millisecondes (et non plus 15 millisecondes) par rapport à la fenêtre précédente. Une fois que l'on a déterminé que l'une de ces fenêtres glissantes plus étroites a fourni un indice au-dessous du seuil limite S, on peut choisir comme instant de fin du pic PEA4 soit l'instant de fin de cette fenêtre, soit celui du début de cette même fenêtre (ce qui permet notamment d'intégrer le délai protosystolique ventriculaire, qui est un laps de temps mécanique complexe durant lequel aucune éjection ventriculaire ne se produit).

Une fois déterminés la présence d'un pic PEA4 et l'instant de fin du pic, cet instant de fin peut être utilisée pour le réglage du DAV, afin que le dispositif ne stimule le ventricule qu'après que la contraction atriale se soit effectivement achevée.

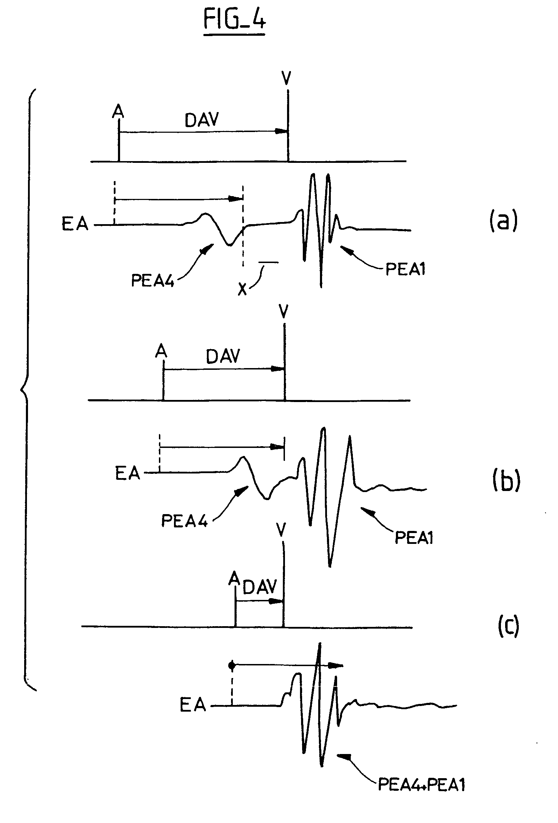

La Figure 4 présente trois exemples de chronogrammes d'un signal EA obtenu, respectivement, dans le cas d'un DAV trop long, d'un DAV correctement ajusté et d'un DAV trop court :
- sur la Figure 4a, on a illustré l'incidence d'un DAV trop long, qui laisse subsister un temps mort X entre la fin de la contraction de l'oreillette (révélée par le PEA4) et le début de contraction du ventricule (résultant de la stimulation V, qui produit sur le signal EA le pic PEA1) ;
- la Figure 4b illustre le cas où le DAV a été ajusté à une valeur optimale, permettant de neutraliser le temps mort X pour déclencher la contraction du ventricule (application de la stimulation V) dès la fin du remplissage de celui-ci, c'est-à-dire dès la fin de la contraction atriale - correspondant sur le signal EA à la fin du pic PEA4 ;
- la Figure 4c illustre une situation où le DAV appliqué présente une valeur trop courte : dans ce cas, le ventricule commence à se contracter avant d'avoir été complètement rempli, diminuant de ce fait le volume éjecté, et donc le débit, de la pompe cardiaque. Sur le signal EA, cette situation est révélée par une fusion du pic PEA4 et du pic PEA1 immédiatement consécutif.

Connaissant l'instant de fin du pic PEA4, il est aisé de donner au DAV une valeur telle que le ventricule ne soit pas stimulé avant la fin du pic PEA4, c'est-à-dire avant la fin de la contraction auriculaire précédant cette stimulation.

Dans la forme de mise en oeuvre la plus simple, le DAV est ajusté à une valeur telle qu'il coïncide avec l'instant de fin du pic PEA4.

Sa valeur peut être déterminée à chaque cycle auriculaire (détecté ou stimulé), ou bien à intervalles réguliers, typiquement une fois par jour, en différenciant éventuellement les événements atriaux stimulés et les événements atriaux spontanés.

En variante, le dispositif peut déterminer le DAV non seulement en fonction de l'instant de fin du PEA4, mais également en fonction de l'instant de début du PEA1 immédiatement consécutif : l'intervalle de temps séparant ces deux instants dépendant de la valeur calculée du DAV, qui peut être déterminée de manière à faire concorder la fin du PEA4 avec le début du PEA1 immédiatement consécutif.

Dans une autre variante encore, le DAV est ajusté de manière que la fin du PEA4 précède d'une durée prédéterminée, typiquement 10 ms, l'instant du début du PEA1 immédiatement consécutif.

## Revendications

1. Un dispositif médical actif implantable du type prothèse cardiaque de stimulation, resynchronisation et/ou défibrillation, comportant :
- des moyens de détection d'événements auriculaires ;
- des moyens de détection d'événements ventriculaires ;
- des moyens pour calculer un délai atrio-ventriculaire (DAV) et pour débuter ce délai atrio-ventriculaire sur détection d'un événement auriculaire spontané ou stimulé, comprenant :
· un capteur d'accélération, apte à délivrer un signal d'accélération endocardiaque (EA) représentatif des mouvements produits par les contractions cycliques de la cavité auriculaire ; et
· des moyens d'analyse de signal d'accélération endocardiaque, aptes à reconnaître et isoler dans le signal d'accélération endocardiaque délivré par le capteur une composante (EA4) correspondant au quatrième pic d'accélération endocardiaque associé à l'activité auriculaire, et à calculer la valeur du délai atrio-ventriculaire à partir de ladite composante (EA4); et
- des moyens de stimulation ventriculaire, aptes à délivrer à l'expiration du délai atrio-ventriculaire une impulsion de faible énergie en l'absence d'événement ventriculaire spontané détecté,
dispositif **caractérisé en ce que** les moyens d'analyse de signal d'accélération endocardiaque sont des moyens aptes à déterminer un paramètre de la composante correspondant au quatrième pic d'accélération endocardiaque associé à l'activité auriculaire fonction de l'instant de fin de ladite composante (EA4), et à calculer la valeur du délai atrio-ventriculaire en fonction dudit paramètre.

2. Le dispositif de la revendication 1 dans lequel la valeur du délai atrio-ventriculaire est calculée de manière que la fin du délai atrio-ventriculaire soit concomitante ou postérieure à l'instant de fin de la composante correspondant au quatrième pic d'accélération endocardiaque associé à l'activité auriculaire (EA4).

3. Le dispositif de la revendication 2, dans lequel la fin du délai atrio-ventriculaire est définie par l'instant de fin de la composante correspondant au quatrième pic d'accélération endocardiaque associé à l'activité auriculaire (EA4).

4. Le dispositif de la revendication 1, dans lequel :
- les moyens d'analyse de signal d'accélération endocardiaque (EA) sont également aptes à reconnaître et isoler dans le signal d'accélération endocardiaque (EA) délivré par le capteur une composante (EA1) correspondant au premier pic d'accélération endocardiaque associé à l'activité auriculaire, et à déterminer un instant de début de cette composante (EA1); et
- la valeur du délai atrio-ventriculaire est également calculée en fonction de l'instant de début de la composante correspondant au premier pic d'accélération endocardiaque associé à l'activité auriculaire (EA1).

5. Le dispositif de la revendication 4, dans lequel la valeur du délai atrio-ventriculaire est calculée en fonction de l'intervalle de temps séparant l'instant de fin de la composante correspondant au quatrième pic d'accélération endocardiaque associé à l'activité auriculaire (EA4) de l'instant de début de la composante correspondant au premier pic d'accélération endocardiaque associé à l'activité auriculaire (EA1) immédiatement consécutive.

6. Le dispositif de la revendication 5, dans lequel la valeur du délai atrio-ventriculaire est calculée de manière à faire concorder l'instant de fin de la composante correspondant au quatrième pic d'accélération endocardiaque associé à l'activité auriculaire (EA4) avec l'instant de début de la composante correspondant au premier pic d'accélartion endocardiaque associé à l'activité auriculaire (EA1) immédiatement consécutive.

7. Le dispositif de la revendication 5, dans lequel la valeur du délai atrio-ventriculaire est calculée de manière que l'instant de fin de la composante correspondant au quatrième pic d'accélération endocardiaque associé à l'activité auriculaire (EA4) précède d'une durée prédéterminée l'instant de début de la composante correspondant au premier pic d'accélération endocardiaque associé à l'activité auriculaire (EA1) immédiatement consécutive.

8. Le dispositif de la revendication 1, dans lequel les moyens d'analyse de signal d'accélération endocardiaque comprennent des moyens pour quantifier un paramètre (I(_{t,t+30)}) du signal d'accélération endocardiaque à l'intérieur d'au moins une fenêtre temporelle d'analyse (Wᵢ) de durée prédéterminée, cette fenêtre étant déclenchée après la détection de l'événement auriculaire (P), et se terminant sur un autre événement spécifique.

9. Le dispositif de la revendication 8, dans lequel ledit autre événement spécifique est un événement du groupe comprenant : détection ventriculaire, stimulation ventriculaire, apparition d'un signal correspondant au premier pic d'accélération endocardiaque associé à l'activité auriculaire (EA1), ou délai fixe.

10. Le dispositif de la revendication 8, dans lequel le paramètre quantifié (I_{(t,t+30)}) est l'énergie du signal d'accélération endocardiaque, intégrée sur la durée de la fenêtre d'analyse (Wᵢ).

11. Le dispositif de la revendication 8, dans lequel les moyens d'analyse de signal d'accélération endocardiaque (EA) comprennent des moyens pour quantifier le paramètre (I_{(t,t+30)}) du signal d'accélération endocardiaque à l'intérieur d'une pluralité de fenêtres temporelles d'analyse successives (W₁ ... Wₙ).

12. Le dispositif de la revendication 11, dans lequel les fenêtres temporelles d'analyse successives (W₁ ... Wₙ) sont des fenêtres chevauchantes.

13. Le dispositif de la revendication 8, dans lequel les moyens d'analyse de signal d'accélération endocardiaque comprennent des moyens comparateurs, pour comparer à un seuil donné (S) le paramètre (I_{(t,t+30)}) du signal d'accélération endocardiaque quantifié à l'intérieur de la fenêtre temporelle (Wᵢ), et définir l'instant de fin de la composante correspondant au quatrième pic d'accélération endocardiaque associé à l'activité auriculaire (EA4) par le franchissement à la baisse de ce seuil.

14. Le dispositif de la revendication 13, dans lequel le seuil (S) est un seuil du groupe formé par : seuil fixe ; seuil paramétrable ; seuil adaptatif recalculé à intervalles réguliers ; seuil adaptatif recalculé sur chaque cycle comprenant un événement auriculaire valide.

15. Le dispositif de la revendication 1, dans lequel le capteur d'accélération est un capteur du groupe formé par : capteur endocavitaire ; capteur épicardique ; capteur externe.

## Claims

1. Implantable active medical device of the stimulation, resynchronization and/or defibrillation cardiac prosthesis type, comprising:
- means for detecting atrial events;
- means for detecting ventricular events;
- means for calculating an atrio-ventricular delay (DAV) and for starting this atrio-ventricular delay upon the detection of a spontaneous or stimulated atrial event, comprising:
· an acceleration sensor, capable of delivering an endocardiac acceleration signal (EA) representative of the movements produced by the cyclical contractions of the atrial cavity; and
· endocardiac acceleration signal analysis means, capable of recognizing and isolating, in the endocardiac acceleration signal delivered by the sensor, a component (EA4) corresponding to the fourth endocardiac acceleration spike associated with the atrial activity, and of calculating the value of the atrio-ventricular delay from said component (EA4); and
- ventricular stimulation means, capable of delivering, on expiry of the atrio-ventricular delay, a pulse of low energy in the absence of any detected spontaneous ventricular event,
the device being **characterized in that** the endocardiac acceleration signal analysis means are means capable of determining a parameter of the component corresponding to the fourth endocardiac acceleration spike associated with the atrial activity that is a function of the end instant of said component (EA4), and of calculating the value of the atrio-ventricular delay as a function of said parameter.

2. Device according to Claim 1, in which the value of the atrio-ventricular delay is calculated so that the end of the atrio-ventricular delay is concomitant with or later than the end instant of the component corresponding to the fourth endocardiac acceleration spike associated with the atrial activity (EA4).

3. Device according to Claim 2, in which the end of the atrio-ventricular delay is defined by the end instant of the component corresponding to the fourth endocardiac acceleration spike associated with the atrial activity (EA4).

4. Device according to Claim 1, in which:
- the means for analysing the endocardiac acceleration signal (EA) are also capable of recognizing and isolating, in the endocardiac acceleration signal (EA) delivered by the sensor, a component (EA1) corresponding to the first endocardiac acceleration spike associated with the atrial activity, and of determining a start instant of said component (EA1); and
- the value of the atrio-ventricular delay is also calculated as a function of the start instant of the component corresponding to the first endocardiac acceleration spike associated with the atrial activity (EA1) .

5. Device according to Claim 4, in which the value of the atrio-ventricular delay is calculated as a function of the time interval between the end instant of the component corresponding to the fourth endocardiac acceleration spike associated with the atrial activity (EA4) and the start instant of the immediately consecutive component corresponding to the first endocardiac acceleration spike associated with the atrial activity (EA1).

6. Device according to Claim 5, in which the value of the atrio-ventricular delay is calculated so as to make the end instant of the component corresponding to the fourth endocardiac acceleration spike associated with the atrial activity (EA4) match the start instant of the immediately consecutive component corresponding to the first endocardiac acceleration spike associated with the atrial activity (EA1).

7. Device according to Claim 5, in which the value of the atrio-ventricular delay is calculated so that the end instant of the component corresponding to the fourth endocardiac acceleration spike associated with the atrial activity (EA4) precedes, by a predetermined time, the start instant of the immediately consecutive component corresponding to the first endocardiac acceleration spike associated with the atrial activity (EA1) .

8. Device according to Claim 1, in which the endocardiac acceleration signal analysis means comprise means for quantizing a parameter (I(ₜ,ₜ₊₃₀₎) of the endocardiac acceleration signal within at least one analysis time window (Wᵢ) of predetermined duration, this window being triggered after the detection of the atrial event (P), and ending on another specific event.

9. Device according to Claim 8, in which said other specific event is an event from the group comprising: ventricular detection, ventricular stimulation, appearance of a signal corresponding to the first endocardiac acceleration spike associated with the atrial activity (EA1), or fixed delay.

10. Device according to Claim 8, in which the quantized parameter (I_{(t,t+30)}) is the energy of the endocardiac acceleration signal integrated over the duration of the analysis window (Wᵢ).

11. Device according to Claim 8, in which the means for analysing the endocardiac acceleration signal (EA) comprise means for quantizing the parameter (I_{(t,t+30)}) of the endocardiac acceleration signal within a plurality of successive analysis time windows (W₁...Wₙ).

12. Device according to Claim 11, in which the successive analysis time windows (W₁...Wₙ) are overlapping windows.

13. Device according to Claim 8, in which the endocardiac acceleration signal analysis means comprise comparator means, for comparing the parameter (I_{(t,t+30)}) of the endocardiac acceleration signal quantized within the time window (Wᵢ) with a given threshold (S), and defining the end instant of the component corresponding to the fourth endocardiac acceleration spike associated with the atrial activity (EA4) by the fact that said parameter has fallen below this threshold.

14. Device according to Claim 13, in which the threshold (S) is a threshold from the group formed by: fixed threshold; configurable threshold; adaptive threshold recalculated at regular intervals; adaptive threshold recalculated on each cycle comprising a valid atrial event.

15. Device according to Claim 1, in which the acceleration sensor is a sensor from the group formed by: endocavitary sensor; epicardiac sensor; external sensor.

## Patentansprüche

1. Implantierbare aktive medizinische Vorrichtung des Typs Stimulations-, Resynchronisations- und/oder Defibrillations-Herzprothese, die umfasst:
- Mittel zum Detektieren atrialer Ereignisse;
- Mittel zum Detektieren ventrikulärer Ereignisse;
- Mittel zum Berechnen einer atrial-ventrikulären Verzögerung (DAV) und zum Beginnen dieser atrial-ventrikulären Verzögerung bei Detektion eines spontanen oder stimulierten atrialen Ereignisses, die umfassen:
· einen Beschleunigungssensor, der dazu ausgelegt ist, ein endokardiales Beschleunigungssignal (EA) zu liefern, das Bewegungen repräsentiert, die durch die zyklischen Kontraktionen des Atriums erzeugt werden; und
· Mittel zum Analysieren des endokardialen Beschleunigungssignals, die dazu ausgelegt sind, in dem endokardialen Beschleunigungssignal, das von dem Sensor geliefert wird, eine Komponente (EA4) zu erkennen und zu isolieren, die der vierten Spitze der endokardialen Beschleunigung entspricht, die der atrialen Aktivität zugeordnet ist, und den Wert der atrial-ventrikulären Verzögerung ausgehend von dieser Komponente (EA4) zu berechnen; und
- Mittel zur ventrikulären Stimulation, die dazu ausgelegt sind, bei Ablauf der atrial-ventrikulären Verzögerung einen Impuls mit geringer Energie bei Abwesenheit eines detektierten spontanen ventrikulären Ereignisses zu verabreichen,
wobei die Vorrichtung **dadurch gekennzeichnet ist, dass** die Mittel zum Analysieren des endokardialen Beschleunigungssignals Mittel sind, die dazu ausgelegt sind, einen Parameter der Komponente, die der vierten Spitze der endokardialen Beschleunigung entspricht, die der atrialen Aktivität zugeordnet ist, als Funktion des Endzeitpunkts dieser Komponente (EA4) zu bestimmen und den Wert der atrial-ventrikulären Verzögerung als Funktion des Parameters zu berechnen.

2. Vorrichtung nach Anspruch 1, wobei der Wert der atrial-ventrikulären Verzögerung in der Weise berechnet wird, dass das Ende der atrial-ventrikulären Verzögerung mit dem Endzeitpunkt der Komponente (EA4), die der vierten Spitze der endokardialen Beschleunigung entspricht und der atrialen Aktivität zugeordnet ist, zusammenfällt oder diesem folgt.

3. Vorrichtung nach Anspruch 2, wobei das Ende der atrial-ventrikulären Verzögerung durch den Endzeitpunkt der Komponente (EA4), die der vierten Spitze der endokardialen Beschleunigung entspricht und der atrialen Aktivität zugeordnet ist, definiert ist.

4. Vorrichtung nach Anspruch 1, wobei:
- die Mittel zum Analysieren des endokardialen Beschleunigungssignals (EA) außerdem dazu ausgelegt sind, in dem endokardialen Beschleunigungssignal (EA), das von dem Sensor geliefert wird, eine Komponente (EA1) zu erkennen und zu isolieren, die der ersten Spitze der endokardialen Beschleunigung entspricht, die der atrialen Aktivität zugeordnet ist, und einen Anfangszeitpunkt dieser Komponente (EA1) zu bestimmen; und
- der Wert der atrial-ventrikulären Verzögerung außerdem als Funktion des Anfangszeitpunkts der Komponente (EA1), die der ersten Spitze der endokardialen Beschleunigung entspricht, die der atrialen Aktivität zugeordnet ist, berechnet wird.

5. Vorrichtung nach Anspruch 4, wobei der Wert der atrial-ventrikulären Verzögerung als Funktion des Zeitintervalls berechnet wird, das den Endzeitpunkt der Komponente (EA4), die der vierten Spitze der endokardialen Beschleunigung entspricht, die der atrialen Aktivität zugeordnet ist, von dem Anfangszeitpunkt der unmittelbar nachfolgenden Komponente (EA1), die der ersten Spitze der endokardialen Beschleunigung entspricht, die der atrialen Aktivität zugeordnet ist, trennt.

6. Vorrichtung nach Anspruch 5, wobei der Wert der atrial-ventrikulären Verzögerung in der Weise berechnet wird, dass der Endzeitpunkt der Komponente (EA4), die der vierten Spitze der endokardialen Beschleunigung entspricht, die der atrialen Aktivität zugeordnet ist, mit dem Anfangszeitpunkt der unmittelbar nachfolgenden Komponente (EA1), die der ersten Spitze der endokardialen Beschleunigung entspricht, die der atrialen Aktivität zugeordnet ist, zur Übereinstimmung gelangt.

7. Vorrichtung nach Anspruch 5, wobei der Wert der atrial-ventrikulären Verzögerung in der Weise berechnet wird, dass der Endzeitpunkt der Komponente (EA4), die der vierten Spitze der endokardialen Beschleunigung entspricht, die der atrialen Aktivität zugeordnet ist, dem Anfangszeitpunkt der unmittelbar nachfolgenden Komponente (EA1), die der ersten Spitze der endokardialen Beschleunigung entspricht, die der atrialen Aktivität zugeordnet ist, um eine vorgegebene Dauer vorhergeht.

8. Vorrichtung nach Anspruch 1, wobei die Mittel zum Analysieren des endokardialen Beschleunigungssignals Mittel umfassen, um einen Parameter (I_{(t,t+30)}) des endokardialen Beschleunigungssignals in wenigstens einem Analysezeitfenster (Wᵢ) mit vorgegebener Dauer zu quantifizieren, wobei dieses Fenster nach der Detektion des atrialen Ereignisses (P) beginnt und bei einem weiteren spezifischen Ereignis endet.

9. Vorrichtung nach Anspruch 8, wobei das weitere spezifische Ereignis ein Ereignis der Gruppe ist, die umfasst: ventrikuläre Detektion, ventrikuläre Stimulation, Auftreten eines Signals (EA1), das der ersten Spitze der endokardialen Beschleunigung entspricht, die der atrialen Aktivität zugeordnet ist, oder feste Verzögerung.

10. Vorrichtung nach Anspruch 8, wobei der quantifizierte Parameter (I_{(t,t+30)}) die Energie des endokardialen Verzögerungssignals ist, die über die Dauer des Analysefensters (Wᵢ) integriert ist.

11. Vorrichtung nach Anspruch 8, wobei die Mittel zum Analysieren des endokardialen Beschleunigungssignals (EA) Mittel umfassen, um den Parameter (I_{(t,t+30)}) des endokardialen Beschleunigungssignals in mehreren aufeinander folgenden Analysezeitfenstern (W₁, ..., Wₙ) zu quantifizieren.

12. Vorrichtung nach Anspruch 11, wobei die aufeinander folgenden Analysezeitfenster (W₁, ..., Wₙ) überlappende Fenster sind.

13. Vorrichtung nach Anspruch 8, wobei die Mittel zum Analysieren des endokardialen Beschleunigungssignals Komparatormittel umfassen, um den Parameter (I_{(t,t+30)}) des quantifizierten endokardialen Beschleunigungssignals in dem Zeitfenster (Wᵢ) mit einem gegebenen Schwellenwert (S) zu vergleichen und den Endzeitpunkt der Komponente (EA4), die der vierten Spitze der endokardialen Beschleunigung entspricht, die der atrialen Aktivität zugeordnet ist, durch das Überschreiten beim Abfall dieses Schwellenwerts zu definieren.

14. Vorrichtung nach Anspruch 13, wobei der Schwellenwert (S) ein Schwellenwert aus der Gruppe ist, die umfasst: fester Schwellenwert; parametrisierbarer Schwellenwert; adaptiver Schwellenwert, der in regelmäßigen Intervallen neu berechnet wird; adaptiver Schwellenwert, der in jedem Zyklus, der ein gültiges atriales Ereignis enthält, neu berechnet wird.

15. Vorrichtung nach Anspruch 1, wobei der Beschleunigungssensor ein Sensor aus der Gruppe ist, die umfasst: einen endokavitären Sensor; einen epikardialen Sensor; einen externen Sensor.
